# EUROPÄISCHE PATENTSCHRIFT

(11) **EP 1 862 154 B1**
(45) Veröffentlichungstag und Bekanntmachung des Hinweises auf die Patenterteilung: **23.03.2011**
(21) Anmeldenummer: 06405242.6
(22) Anmeldetag: 31.05.2006
(51) Int. Cl.: A61F 9/01

(54) **Ophtalmologische Vorrichtung**
Ophthalmologic Device
Dispositif ophthalmologique

(43) Veröffentlichungstag der Anmeldung: 05.12.2007
(73) Patentinhaber: Ziemer Holding AG, 2562 Port (CH)
(72) Erfinder: Rathjen, Christian, 28197 Bremen (DE)
(74) Vertreter: Vogel, Dany

(56) Entgegenhaltungen:
- EP-A1- 1 486 185
- WO-A-98/48746
- WO-A-2005/092259
- US-A- 4 973 330
- US-A- 5 993 438
- US-A1- 2002 035 359
- US-B1- 6 648 877

## Beschreibung

### Technisches Gebiet

Die vorliegende Erfindung betrifft eine ophthalmologische Vorrichtung mit einem Lichtprojektor zur Projektion von Femtosekundenlaserpulsen für die Auflösung von Augengewebe. Die vorliegende Erfindung betrifft insbesondere eine ophthalmologische Vorrichtung mit einem Lichtprojektor zur Projektion von Femtosekundenlaserpulsen für die Auflösung von Augengewebe, wobei die Femtosekundenlaserpulse jeweils auf eine Fokalfläche projiziert werden.

### Stand der Technik

Bei Femtolasersystemen, die Pulsbreiten von typisch 10fs bis 1000fs (1fs=10⁻¹⁵s) aufweisen, besteht die Besonderheit, dass auch transparente Materialen im Fokus durch nichtlineare Absorption und anschliessende Wechselwirkung (z.B. Photodisruption) bearbeitet werden können. Bezeichnend für Femtolasersysteme ist die Möglichkeit der freien Fokusführung im Material, was die Erzeugung beliebiger Schnittmuster ermöglicht. Als Beispiel sei insbesondere das in die Praxis eingeführte operative Schneiden in der Augenhomhaut (Cornea) angeführt, wofür mehrere Laserpulse mittels einer Abtastvorrichtung (Scanner) hinter respektive nebeneinander gereiht werden. Um bei Schneideoperationen verbleibende Gewebebrücken zu vermeiden, werden die einzelnen Laserpulse durch die Abtastvorrichtung so positioniert, dass sich die Bearbeitungsbereiche von nacheinander folgenden Laserpulsen überlappen. Die durch einen nachfolgenden Laserpuls in den Überlappungsbereich eingestrahlte Energie trägt allerdings nicht zur für den Schnitt gewünschten Gewebeauflösung bei. Die vom nachfolgenden Laserpuls im Überlappungsbereich deponierte Energie geht ungenutzt verloren und/oder belastet auf unerwünschte Weise (Erhitzung) an den Schnitt angrenzendes Gewebe.

In der Patentanmeldung WO 2005/092259 wird eine Vorrichtung zur Materialbearbeitung mittels nicht-linearer Laserstrahlung beschrieben. Nach WO 2005/092259 wird zur Vermeidung von Kollateralschäden in benachbarten Materialbereichen die Fokusgrösse mittels eines Polarisationsmodulators und/oder eines Intensitätsmodulators im Strahlengang verringert. Der oben angeführte Nachteil bei überlappenden Bearbeitungsbereichen von nacheinander folgenden Laserpulsen bleibt jedoch bestehen.

Im Patent US 5,993,438 wird eine ophthalmologische Vorrichtung für die intrastromale photorefraktive Keratektomie mittels Femtosekundenlaserpulsen beschrieben. In der Vorrichtung nach US 5,993,438 sind die fokalen Pulsbearbeitungsbereiche ("Focal Spots") im Wesentlichen kreisrund oder weisen eine leicht elliptische Form auf. Um "Focal Spots" mit einer möglichst kreisrunden statt elliptischen Form zu erhalten, wird der Laserstrahl durch einen breiten Kegelwinkel fokussiert.

Eine weitere ophthalmologische Vorrichtung, welche Femtosekundenlaserpulse für die Bearbeitung von Augengewebe verwendet, wird in der Patentschrift US 6,648,877 beschrieben.

In der Patentanmeldung WO 98/48746 wird ein Excimerlasersystem beschrieben, welches in zwei verschiedenen Modi mit Spotgrössen unterschiedlicher Durchmesser betrieben werden kann.

### Darstellung der Erfindung

Es ist eine Aufgabe der vorliegenden Erfindung eine ophthalmologische Vorrichtung mit einem Lichtprojektor zur Projektion von Femtosekundenlaserpulsen für die Auflösung von Augengewebe vorzuschlagen, welche nicht die Nachteile des Stands der Technik aufweist. Es ist insbesondere eine Aufgabe der vorliegenden Erfindung eine ophthalmologische Vorrichtung mit einem Lichtprojektor zur Projektion von Femtosekundenlaserpulsen für die Auflösung von Augengewebe vorzuschlagen, welche ermöglicht die unerwünschte Energieeinstrahlung in Überlappungsgebiete nacheinander folgender Laserpulse zu verringern, ohne dadurch verbleibende Gewebebrücken zu erhalten.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass eine ophthalmologische Vorrichtung, welche einen Lichtprojektor umfasst zur Projektion von Femtosekundenlaserpulsen für eine Auflösung von Augengewebe, jeweils fokussiert auf eine Fokalfläche (z.B. eine Fokalebene oder eine konvexe Fokalfläche), mit optischen Mitteln versehen ist zur Erzeugung von jeweils durch einen der Femtosekundenfaserpulse auf der Fokalfläche gebildeten Pulsbearbeitungsbereichen mit jeweils einer von einer kreisrunden Form abweichenden Bereichsgrenze auf der Fokalfläche. Die ophthalmologische Vorrichtung ist beispielsweise eingerichtet zur Erzeugung von Schnitten im Augengewebe. Die Bereichsgrenze ist beispielsweise definiert durch einen Grenzwert für den Intensitätsabfall eines der Laserpulse auf der Fokalfläche, d.h. die Bereichsgrenze eines Pulsbearbeitungsbereichs wird dort festgelegt, wo die Intensität des eingestrahlten Lichts auf der Fokalfläche um oder auf mindestens den definierten Grenzwert abfällt, zum Beispiel um 60% respektive auf 40%. Die erzeugten Pulsbearbeitungsbereiche haben beispielsweise eine elliptisch oder oval geformte Bereichsgrenze und weisen vorzugsweise in einer Längsrichtung eine breitere Ausdehnung auf als in einer zur Längsrichtung normalen Querrichtung mit einer schmäleren Ausdehnung. Die erzeugten Pulsbearbeitungsbereiche sind im Vergleich zu den herkömmlichen kreisrunden Pulsbearbeitungsbereichen vorzugsweise in der Längsrichtung gestreckt und in der Querrichtung gestaucht. Im Vergleich zu herkömmlichen kreisrunden Pulsbearbeitungsbereichen können die erfindungsgemäss erzeugten Pulsbearbeitungsbereiche bezüglich einer Bearbeitungslinie so ausgerichtet werden, dass bei gleichem Pulsabstand durch nacheinander folgende Pulsbearbeitungsbereiche kleinere Überlappungsbereiche entstehen. Somit wird die unerwünschte Energieeinstrahlung in Überlappungsgebiete nacheinander folgender Laserpulse verringert, ohne dass dadurch Gewebebrücken verbleiben. Zudem können die erfindungsgemäss erzeugten Pulsbearbeitungsbereiche bezüglich der Bearbeitungsrichtung so ausgerichtet werden, dass bei gleichem Pulsabstand, eine breitere Schnittspur erzeugt wird. Bei der Erzeugung von Schnittflächen durch mehrere aneinander gereihte Schnittspuren kann somit die Gesemtbearbeitungszeit (Schnittzeit) reduziert werden. Zudem wird auch die bei der Augenbehandlung insgesamt ins Augengewebe eingestrahlte Energie reduziert.

Vorzugsweise werden die Pulsbearbeitungsbereiche durch Positionierungsmittel entlang der Bearbeitungslinie positioniert und die optischen Mittel sind eingerichtet, die Pulsbearbeitungsbereiche bezüglich der Bearbeitungslinie auszurichten. Bei geraden Bearbeitungslinien sind die optischen Mittel zur Ausrichtung der Pulsbearbeitungsbereiche bezüglich der Bearbeitungslinie fest eingestellt. Bei gekrümmten Bearbeitungslinien, z.B. kreis- oder spiralförmig, wird die Einstellung der optischen Mittel zur Ausrichtung der Pulsbearbeitungsbereiche bezüglich der Bearbeitungslinie dynamisch verändert. Die optischen Mittel sind beispielsweise eingerichtet, die Pulsbearbeitungsbereiche bezüglich der Bearbeitungslinie so auszurichten (fest oder veränderlich), dass eine in der breiteren Ausdehnung des Pulsbearbeitungsbereichs verlaufende Längsachse jeweils senkrecht zur Bearbeitungslinie liegt. Durch die Querstellung der gestreckten Ausdehnung der Pulsbearbeitungsbereiche zur Bearbeitungslinie entstehen, wie oben angeführt, kleinere Überlappungsbereiche der Pulsbearbeitungsbereiche nacheinander folgender Laserpulse, wodurch unerwünschte Energieeinstrahlung in Überlappungsgebiete verringert wird, ohne dass dadurch Gewebebrücken verbleiben. Überdies kann eine breitere Schnittspur erzeugt werden, wodurch die Schnittzeit für eine Schnittfläche reduziert wird. Die optischen Mittel sind beispielsweise als Alternative oder zusätzlich eingerichtet, die Pulsbearbeitungsbereiche bezüglich der Bearbeitungslinie so auszurichten (fest oder veränderlich), dass eine in der schmäleren Ausdehnung des Pulsbearbeitungsbereichs verlaufende Querachse jeweils senkrecht zur Bearbeitungslinie liegt. Durch die Längsausrichtung der gestreckten Ausdehnung der Pulsbearbeitungsbereiche zur Bearbeitungslinie können verbleibende Gewebebrücken zwischen Pulsbearbeitungsbereichen nacheinander folgender Laserpulse vermieden werden, wenn beispielsweise die Ablenkgeschwindigkeit eines Scanners der Positionierungsmittel zu hoch für die Pulsrate des Lasers ist, der die Femtosekundenlaserpulse erzeugt. Zudem kann die Längsausrichtung der gestreckten Ausdehnung der Pulsbearbeitungsbereiche aber auch die Querausrichtung der gestauchten Ausdehnung der Pulsbearbeitungsbereiche in alternativen Anwendungen eingesetzt werden, in denen eine starke Überlappung aufeinander folgender Pulsbearbeitungsbereiche gewünscht wird, beispielsweise Bearbeitungsverfahren, die mehr als einen Laserpuls auf die gleiche Stelle setzen, um Gewebe abzutragen. Solche Verfahren arbeiten mit sehr hohen Pulsraten (MHz) und geringeren Pulsenergien um einen akkumulierten Effekt von zeitlich aufeinander folgenden Laserpulsen zu nutzen. Durch die Erzeugung von Pulsbearbeitungsbereichen mit einer von einer kreisrunden Form abweichenden Bereichsgrenze und durch die gezielte Ausrichtung der Pulsbearbeitungsbereiche bezüglich der Bearbeitungslinie können Laser und Positionierungsmittel, insbesondere Scannermodule, besser aufeinander abgestimmt bzw. optimierter eingesetzt werden, was beispielsweise dann von Vorteil ist, wenn die Frequenz einer Femtolaserquelle nicht oder nur in ganzzahligen Verhältnissen (durch Pulsselektion) zu seiner Grundfrequenz einstellbar ist.

in einer Ausführungsvariante umfasst die ophthalmologische Vorrichtung ein Steuermodul, das eingerichtet ist, die optischen Mittel derart zu steuern, dass die optischen Mittel die Pulsbearbeitungsbereiche bezüglich der Bearbeitungslinie entsprechend einem Steuerwert ausrichten und/oder die Form der Bereichsgrenze entsprechend dem Steuerwert erzeugen. Eine derartige Steuerung ermöglicht die Form und Ausrichtung der Pulsbearbeitungsbereiche, und/oder die Grösse der Überlappungsbereiche respektive einen Überlappungsgrad flexibel einzustellen.

Vorzugsweise sind die Positionierungsmittel und die optischen Mittel eingerichtet, die Pulsbearbeitungsbereiche so zu positionieren respektive zu erzeugen, dass sich nacheinander folgende Pulsbearbeitungsbereiche mindestens teilweise überlappen, so dass zwischen aufeinander folgenden Pulsbearbeitungsbereichen keine unerwünschten Gewebebrücken verbleiben.

Vorzugsweise umfassen die optischen Mittel zur Erzeugung der Pulsbearbeitungsbereiche einen optischen Modulator, insbesondere einen Polarisationsmodulator, einen Phasenmodulator und/oder einen Intensitätsmodulator, d.h. einen optischen Modulator zur Modulation der Strahlungsintensität, der Phasenlaufzeiten und/oder der Polarität über den Strahlquerschnitt der Femtosekundenlaserpulse. Die optischen Mittel zur Erzeugung der Pulsbearbeitungsbereiche umfassen beispielsweise ein oder mehrere drehbare optische Elemente, z.B. anamorphotische Optikmodule, deformierbare Spiegel, photonische Kristalle, photonische "band-gap" Fasem, diffraktive Optikmodule, Linsen-Arrays, Polarisationsfilter, räumliche Polarisationsplatten, λ-Halbe-Platten, Blenden, und/oder einen oder mehrere räumliche Lichtmodulatoren, z.B. LCD-Arrays oder DLP-Projektoren (Digital Light Processing) (Spiegel-Artays),.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
Figur 1 zeigt ein Blockdiagramm, welches schematisch eine ophthalmologische Vorrichtung bei der Behandlung eines Auges mittels eines fokussierten gepulsten Laserstrahls darstellt.
Figuren 2a, 2b, 2c und 2d zeigen jeweils ein Blockdiagramm, welches schematisch eine Ausführungsvariante der ophthalmologischen Vorrichtung bei der Behandlung eines Auges mittels eines fokussierten gepulsten Laserstrahls darstellt.
Figur 3a zeigt einen durch einen Femtosekundenlaserpuls auf der Fokalfläche gebildeten Pulsbearbeitungsbereich mit einer kreisrunden Bereichsgrenze gemäss dem Stand der Technik.
Figur 3b zeigt mehrere entlang einer Bearbeitungslinie aufeinander folgende Pulsbearbeitungsbereiche mit jeweils einer kreisrunden Bereichsgrenze gemäss dem Stand der Technik.
Figur 4a zeigt einen durch einen Femtosekundenlaserpuls auf der Fokalfläche gebildeten Pulsbearbeitungsbereich mit einer von der kreisrunden Form abweichenden Bereichsgrenze.
Figur 4b zeigt mehrere entlang einer geraden Bearbeitungslinie aufeinander folgende Pulsbearbeitungsbereiche mit einer von der kreisrunden Form abweichenden Bereichsgrenze und mit einer Längsausrichtung quer zur Bearbeitungslinie.
Figur 4c zeigt eine Ausrichtung eines Pulsbearbeitungsbereichs mit einer von der kreisrunden Form abweichenden Bereichsgrenze entlang einer gekrümmten Bearbeitungslinie.
Figur 5a zeigt mehrere entlang einer geraden Bearbeitungslinie aufeinander folgende Pulsbearbeitungsbereiche mit einer von der kreisrunden Form abweichenden Bereichsgrenze und mit einer Längsausrichtung entlang der Bearbeitungslinie.
Figur 5b zeigt eine weitere Ausrichtung eines Pulsbearbeitungsbereichs mit einer von der kreisrunden Form abweichenden Bereichsgrenze entlang der gekrümmten Bearbeitungslinie.
Figur 6 zeigt mehrere aneinander gereihte Schnittspuren, die jeweils durch entlang der geraden Bearbeitungslinie aufeinander folgende Pulsbearbeitungsbereiche gebildet werden.

### Wege zur Ausführung der Erfindung

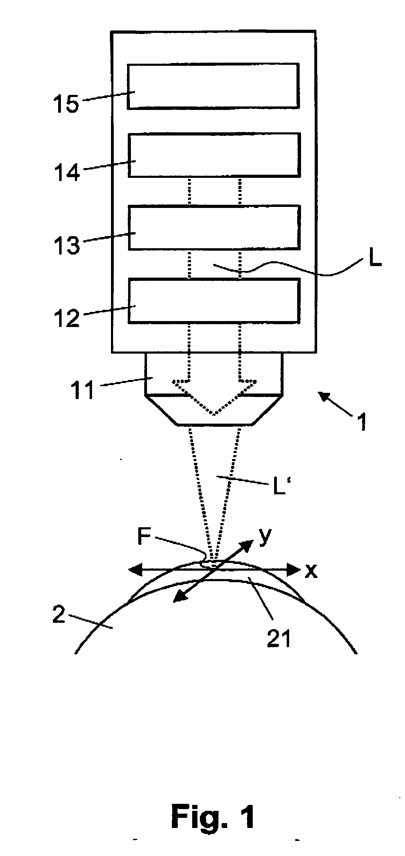

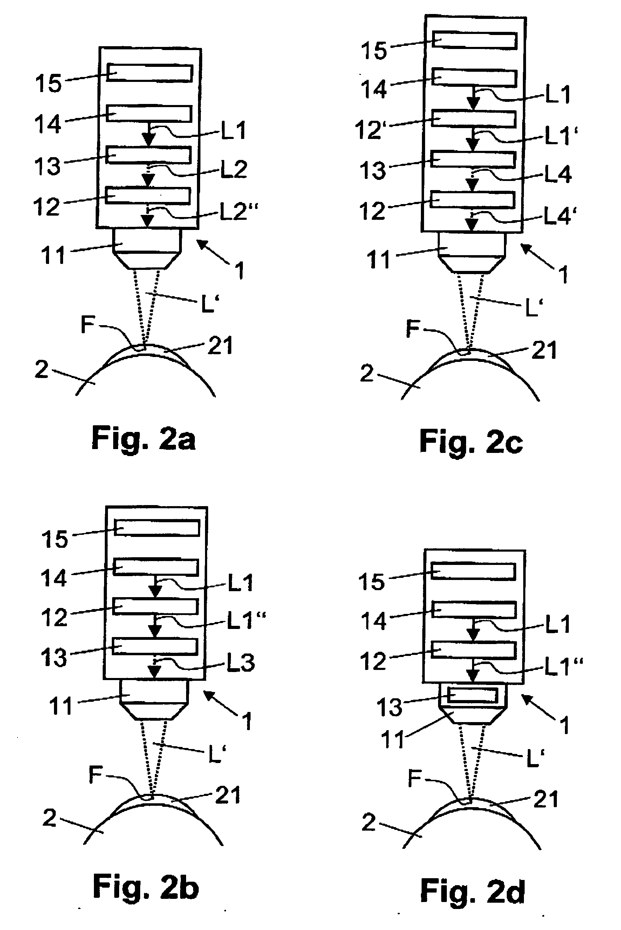

In den Figuren 1, 2a, 2b, 2c und 2d bezeichnet das Bezugszeichen 1 eine ophthalmologische Vorrichtung mit einer Laserquelle 14 und einem mit der Laserquelle 14 optisch verbundenen Lichtprojektor 11 zur Erzeugung und fokussierten Projektion eines gepulsten Laserstrahls L' für die punktuelle Gewebeauflösung in einem Fokus F (Brennpunkt) im Innem des Augengewebes 21, beispielsweise in der Hornhaut (Cornea). Die Laserquelle 14 umfasst einen Femtolaser zur Erzeugung von Femtosekundenlaserpulsen, die Pulsbreiten von typisch 10fs bis 1000fs (1fs=10⁻¹⁵s) aufweisen. Die Laserquelle 14 ist in einem separaten oder in einem mit dem Lichtprojektor 11 gemeinsamen Gehäuse angeordnet. Wie in der Figur 1 schematisch dargestellt ist, umfasst die ophthalmologische Vorrichtung 1 Positionierungsmittel 12 um den Fokus F des gepulsten Laserstrahls L' mindestens in zwei Dimensionen x, y eines (zusammenhängenden oder nicht zusammenhängenden) Bearbeitungsgebiets im oder auf dem Gewebe des Auges 3 zu bewegen. In einer Ausführungsvariante sind die Positionierungsmittel 12 zudem eingerichtet, den Fokus F auch in einer dritten, zu den zwei Dimensionen x, y normalen Richtung zu bewegen. In einer Ausführungsvariante wird die Positionierung und Bewegung des Fokus F einzig durch ein optisches Abtastmodul (Scannermodul) bewirkt, welches den von der Laserquelle 14 erzeugten gepulsten Lichtstrahl entsprechend ablenkt. Die Positionierungsmittel 12 umfassen in einer Ausführungsvariante zusätzlich zum optischen Abtastmodul einen oder mehrere Bewegungstreiber zur Bewegung des Lichtprojektors 11. Die Positionierungsmittel 12 sind beispielsweise wie in der per Referenz miteingeschlossenen EP 1 486 185 ausgeführt und überlagern in einer Ausführungsvariante den durch Bewegung des Lichtprojektors 11 verursachten translatorischen Bewegungen des Fokus gemäss EP 1 627 617 eine zusätzliche feine Bewegung mittels optischer Microscans. In der noch nicht veröffentlichten Europäischen Patentanmeldung Nr. 05 405 376 werden ein Scannermodul zur Ablenkung des gepulsten Lichtstrahls für die zusätzliche feine Bewegung, sowie ein optisches Übertragungssystem zur Übertragung der abgelenkten Femtosekundenlaserpulse vom Scannermodul zum Lichtprojektor 11 und zur Überlagerung der abgelenkten Femtosekundenlaserpulse auf die Bewegung des Lichtprojektors 11 beschrieben.

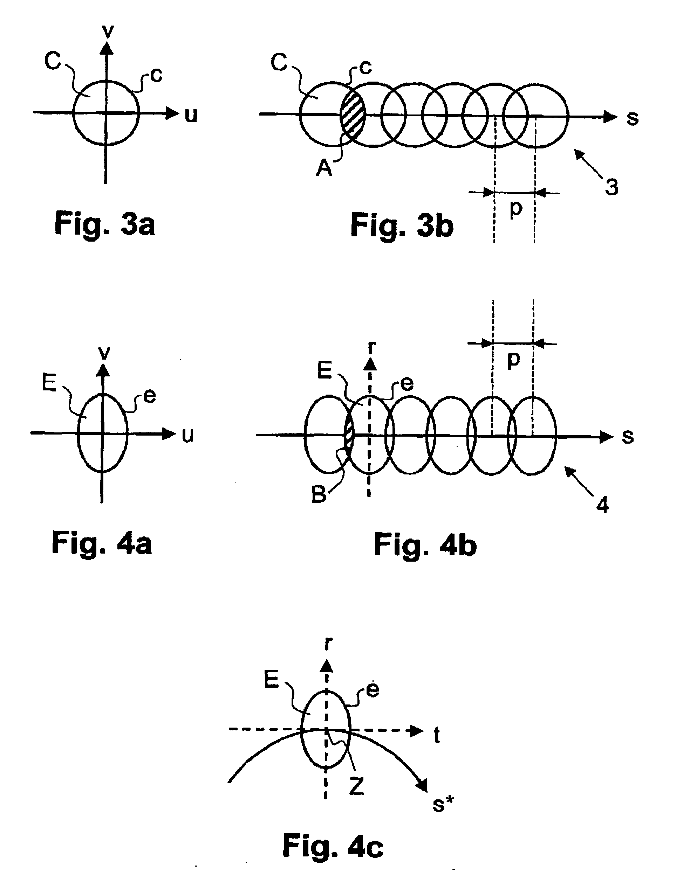

Durch die fokussierte Projektion des gepulsten Laserstrahls L' werden durch die Femtosekundenlaserpulse jeweils auf der Fokalfläche (d.h. Brennebene oder konvexe Brennfläche) Pulsbearbeitungsbereiche gebildet. Die Bereichsgrenze eines Pulsbearbeiturtgsbereichs ist beispielsweise definiert durch einen Grenzwert G für den Intensitätsabfall 1/e² des auf der Fokalfläche fokussierten Femtosekundenlaserpulses. Die Bereichsgrenze ist beispielsweise dort definiert, wo die Intensität des eingestrahlten Lichts auf der Fokalfläche um oder auf mindestens den definierten Grenzwert G abfällt, zum Beispiel um 60% respektive auf 40%. In den Figuren 3a und 4a bezeichnen die Bezugszeichen u und v die Koordinatenachsen der mit der Zeichenebene zusammenfallenden Fokalfläche. Wie in der Figur 3a dargestellt ist, weist ein durch einen Femtosekundenlaserpuls eines bekannten Systems erzeugter Pulsbearbeitungsbereich C auf der Fokalfläche eine kreisrunde Bereichsgrenze c auf. Die Figur 3b zeigt eine Schnittspur 3, welche mehrere entlang der geraden Bearbeitungslinie s aufeinander folgende Pulsbearbeitungsbereiche C aufweist. Wie aus der Figur 3b ersichtlich ist, weisen die Pulsbearbeitungsbereiche C, die durch aufeinander folgende Femtosekundenlaserpulse gebildet werden, Überlappungsbereiche A auf.

Wie in der Figur 1 dargestellt ist, umfasst die ophthalmologische Vorrichtung 1 zudem optische Mittel 13, die im schematisch dargestellten Strahlengang L zwischen Laserquelle 14 und Austritt des Lichtprojektors 11 angeordnet sind. Die optischen Mittel 13 sind eingerichtet, die Strahlungsintensität, die Phasenlaufzeiten und/oder Polarität über den Strahlquerschnitt der Femtosekundenlaserpulse so zu beeinflussen, dass die Femtosekundenlaserpulse auf der Fokalfläche Pulsbearbeitungsbereiche E mit einer von der kreisrunden Form abweichenden Bereichsgrenze e bilden, wie beispielsweise in der Figur 4a dargestellt ist. Die durch die optischen Mittel 13 erzeugten Pulsbearbeitungsbereiche E sind im Vergleich zu den herkömmlichen kreisrunden Pulsbearbeitungsbereichen C vorzugsweise in einer Längsrichtung gestreckt und in einer Querrichtung gestaucht und weisen beispielsweise eine elliptische oder oval geformte Bereichsgrenze e auf. In einer Ausführungsvariante ist die Laserquelle 14 eingerichtet, Femtosekundenlaserpulse mit einer von der (durch typisches Gaussprofil bedingten) kreisrunden Form abweichenden elliptisch oder oval geformten Bereichsgrenze e zu erzeugen. In dieser letztgenannten Ausführungsvariante sind die optischen Mittel 13 bloss eingerichtet, die von der Laserquelle 14 erzeugten Femtosekundenlaserpulse über den Lichtprojektor 11 fokussiert auf die Fokalfläche abzubilden respektive zu projizieren, um auf der Fokalfläche Pulsbearbeitungsbereiche E mit einer elliptisch oder oval geformten Bereichsgrenze (e) zu erzeugen.

Zur Erzeugung der von der kreisrunden Form abweichenden Pulsbearbeitungsbereiche E umfassen die optischen Mittel 13 einen Polarisationsmodulator, einen Phasenmodulator und/oder einen Intensitätsmodulatar zur Modulation der Strahlungsintensität, der Phasenlaufzeiten und/oder der Polarität über den Strahlquerschnitt der Femtosekundenlaserpulse. Die optischen Mittel 13 umfassen beispielsweise ein anamorphotisches Optikmodul (z.B. bewegliche sphärische und/oder zylindrische Linsen), einen oder mehrere deformierbare Spiegel, photonische Kristalle, photonische "band-gap" Fasern, diffraktive Optikmodule, Linsen-Arrays, Polarisationsfilter, räumliche Polarisationsplatten, λ-Halbe-Platten, Blenden, und/oder einen oder mehrere räumliche Lichtmodulatoren, z.B. LCD-Arrays oder DLP-Projektoren. In der Ausführungsvariante nach Figur 2a sind die optischen Mittel 13 als Optikmodul in den Strahlengang zwischen der Laserquelle 14 und den Positionierungsmifteln 12 eingefügt. Die von der Laserquelle 14 erzeugten Femtosekundenlaserpulse L1 werden den optischen Mitteln 13 zugeführt, welche die Strahlungsintensität, die Phasenlaufzeiten und/oder die Polarität über den Strahlquerschnitt der Femtosekundenlaserpulse L1 zur Erzeugung nicht-kreisrunder Pulsbearbeitungsbereiche E modulieren. Die von den optischen Mitteln 13 erzeugten Femtosekundenlaserpulse L2 mit modulierter Strahlungsintensität, Phasenlaufzeit und/oder Polarität werden den Positionierungsmitteln 12 zugeführt. Die Positionierungsmittel 12 lenken die Femtosekundenlaserpulse L2 in mindestens eine Abtastrichtung ab und/oder bewegen den Lichtprojektor 11 in eine oder zwei weitere Abtastrichtungen. Die von den Positionierungsmitteln 12 abgelenkten Femtosekundenlaserpulse L2" werden dem Lichtprojektor 11 zur fokussierten Projektion zugeführt.

In der Ausführungsvariante nach Figur 2b sind die optischen Mittel 13 als Optikmodul in den Strahlengang zwischen den Positionierungsmitteln 12 und dem Lichtprojektor 11 eingefügt. Die von der Laserquelle 14 erzeugten Femtosekundenlaserpulse L1 werden den Positionierungsmitteln 12 zugeführt, welche die Femtosekundentaserpulse L1 in mindestens eine Abtastrichtung ablenken und/oder den Lichtprojektor 11 in eine oder zwei weitere Abtastrichtungen bewegen. Die von den Positionierungsmitteln 12 abgelenkten Femtosekundenlaserpulse L1" werden den optischen Mitteln 13 zugeführt, welche die Strahlungsintensität, die Phasenlaufzeit und/oder die Polarität über den Strahlquerschnitt der Femtosekundenlaserpulse L1" zur Erzeugung nicht-kreisrunder Pulsbearbeitungsbereiche E modulieren. Die von den optischen Mitteln 13 erzeugten Femtosekundenlaserpulse L3 mit modulierter Strahlungsintensität, Phasenlaufzeit und/oder Polarität werden dem Lichtprojektor 11 zur fokussierten Projektion zugeführt.

In der Ausführungsvariante nach Figur 2c sind die optischen Mittel 13 als Optikmodul in den Strahlengang zwischen zwei Modulen der Positionierungsmittel 12, 12' eingefügt. Die von der Laserquelle 14 erzeugten Femtosekundenlaserpulse L1 werden dem ersten Modul der Positionierungsmittel 12' zugeführt, welche die Femtosekundenlaserpulse L1 in mindestens eine Abtastrichtung ablenken. Die von den Positionierungsmitteln 12 abgelenkten Femtosekundenlaserpulse L1' werden den optischen Mitteln 13 zugeführt, welche die Strahlungsintensität, Phasenlaufzeit und/oder die Polarität über den Strahlquerschnitt der Femtosekundenlaserpulse L1' zur Erzeugung nicht-kreisrunder Pulsbearbeitungsbereiche E modulieren. Die von den optischen Mitteln 13 erzeugten Femtosekundenlaserpulse L4 mit modulierter Strahlungsintensität, Phasenlaufzeit und/oder Polarität werden dem zweiten Modul der Positionierungsmittel 12 zugeführt, welche die Femtosekundenlaserpulse L4 in mindestens eine weitere Abtastrichtung ablenken und/oder den Lichtprojektor 11 in eine oder zwei weitere Abtastrichtungen bewegen. Die vom zweiten Modul der Positionierungsmittel 12 abgelenkten Femtosekundenlaserpulse L4' werden dem Lichtprojektor 11 zur fokussierten Projektion zugeführt.

In der Ausführungsvariante nach Figur 2d sind die optischen Mittel 13 in den Lichtprojektor 11 integriert. Die von der Laserquelle 14 erzeugten Femtosekundenlaserpulse L1 werden den Positionierungsmitteln 12 zugeführt, welche die Femtosekundenlaserpulse L1 in mindestens eine Abtastrichtung ablenken und/oder den Lichtprojektor 11 in eine oder zwei weitere Abtastrichtungen bewegen. Die von den Positionierungsmitteln 12 abgelenkten Femtosekundenlaserpulse L1" werden den optischen Mitteln 13 im Lichtprojektor 11 zugeführt, welche die Strahlungsintensität, die Phasenlaufzeit und/oder die Polarität über den Strahlquerschnitt der Femtosekundenlaserpulse L1" zur Erzeugung nicht-kreisrunder Pufsbearbeitungsbereiche E modulieren. Die von den optischen Mitteln 13 erzeugten Femtosekundenlaserpulse mit modulierter Strahlungsintensität, Phasenlaufzeit und/oder Polarität werden vom Lichtprojektor 11 fokussiert projiziert.

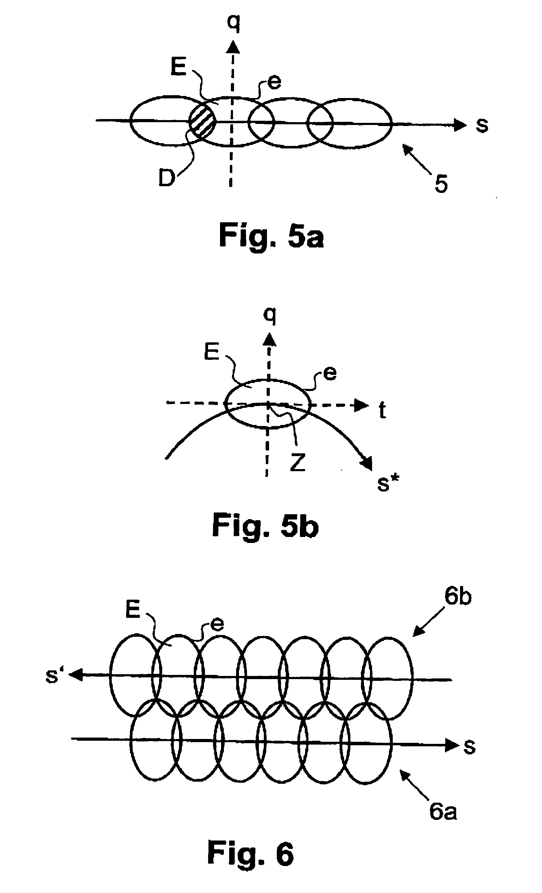

In den Figuren 1, 2a, 2b, 2c und 2d bezeichnet das Bezugszeichen 15 ein Steuermodul, welches als programmiertes Logikmodul mittels Software und/oder Hardware ausgeführt ist. Das Steuermodul 15 ist eingerichtet die Positionierungsmittel 12 und die optischen Mittel 13 zu steuern. Das Steuermodul 15 ist insbesondere eingerichtet, die optischen Mittel 13 zur Ausrichtung der Pulsbearbeitungsbereiche E bezüglich der Bearbeitungslinie s zu steuern. In einer Ausführungsvariante ist das Steuermodul 15 überdies eingerichtet, die optischen Mittel 13 zur Erzeugung von Pulsbearbeitüngsbereichen E mit einer unterschiedlichen, von der Kreisform abweichenden Form zu steuern. Die Steuerung der optischen Mittel 13 hinsichtlich der Ausrichtung und/oder Form der Pulsbearbeitungsbereiche E basiert vorzugsweise auf einem oder mehreren benutzerspezifischen Steuerwerten, welche die Form der Pulsbearbeitungsbereiche E, die Grösse der Überlappungsbereiche B, D (siehe Figur 5a), respektive einen Überlappungsgrad, und/oder das Bearbeitungsmuster definieren.

Die Figur 4b zeigt eine Schnittspur 4, welche mehrere entlang der geraden Bearbeitungslinie s aufeinander folgende Pulsbearbeftungsbereiche E aufweist, die eine von der kreisrunden Form abweichenden Bereichsgrenze e gemäss Figur 4a haben und so ausgerichtet sind, dass eine in der breiteren Ausdehnung des Pulsbearbeitungsbereichs E verlaufende Längsachse r jeweils senkrecht zur Bearbeitungslinie s liegt. Wie aus der Figur 4b ersichtlich ist, weisen die Pulsbearbeitungsbereiche E, die durch aufeinander folgende Femtosekundenlaserpulse mit gleichem Pulsabstand p wie in der Figur 3b gebildet werden, kleinere Überlappungsbereiche B auf als die kreisrunden Pulsbearbeitungsbereiche C in der Figur 3b.

Die Figur 4c zeigt eine Ausrichtung eines Pulsbearbeitungsbereichs E, der eine Bereichsgrenze e gemäss Figur 4a aufweist, entlang einer gekrümmten Bearbeitungslinie s*. Wie aus der Figur 4c ersichtlich ist, ist der Pulsbearbeitungsbereich E so ausgerichtet, dass die in der breiteren Ausdehnung des Pulsbearbeitungsbereichs E verlaufende Längsachse r senkrecht zur Tangente t der gekrümmten Bearbeitungslinie s* liegt, wobei der Zentrumspunkt Z des Pulsbearbeitungsbereichs E dem Berührungspunkt der Tagente t entspricht.

Die Figur 5a zeigt eine Schnittspur 5, welche mehrere entlang der geraden Bearbeitungslinie s aufeinander folgende Pulsbearbeitungsbereiche E aufweist, die eine von der kreisrunden Form abweichenden Bereichsgrenze e gemäss Figur 4a haben und so ausgerichtet sind, dass eine in der schmäleren Ausdehnung des Pulsbearbeitungsbereichs E verlaufende Querachse q jeweils senkrecht zur Bearbeitungslinie s liegt.

Die Figur 5b zeigt eine Ausrichtung eines Pulsbearbeitungsbereichs E, der eine Bereichsgrenze e gemäss Figur 4a aufweist, entlang einer gekrümmten Bearbeitungslinie s*. Wie aus der Figur 5b ersichtlich ist, ist der Pulsbearbeitungsbereich E so ausgerichtet, dass die in der schmäleren Ausdehnung des Pulsbearbeitungsbereichs E verlaufende Querachse q senkrecht zur Tangente t der gekrümmten Bearbeitungslinie s* liegt, wobei der Zentrumspunkt Z des Pulsbearbeitungsbereichs E dem Berührungspunkt der Tagente t entspricht.

Figur 6 zeigt mehrere aneinander gereihte Schnittspuren 6a, 6b gemäss der Figur 4b. Wie aus der Figur 6 ersichtlich ist, sind die Pulsbearbeitungsbereiche E der ersten Schnittspur 6a, entlang der Bearbeitungslinie s, und die Pulsbearbeitungsbereiche E der zweiten Schnittspur 6a, entlang der Bearbeitungslinie s', so phasenverschoben, dass keine Gewebebrücken zwischen den Schnittspuren 6a, 6b verbleiben und möglichst geringe Überlappungsbereiche durch die Pulsbearbeitungsbereiche E benachbarter Schnittspuren 6a, 6b gebildet werden.

Abschliessend soll festgehalten werden, dass die beschriebene ophthalmologische Vorrichtung 1 eine Auflösung von Augengewebe als dreidimensionaler Abtragprozess ermöglicht, wobei die in den Figuren 3a, 3b, 4a, 4b, 4c, 5a, 5b und 6 dargestellten Effekte jeweils in der Aufsicht dargestellt sind.

## Patentansprüche

1. Ophthalmologische Vorrichtung (1) zum Erzeugung von Schnitten im Augengewebe (21), umfassend:
einen Lichtprojektor (11) zur Projektion von Femtosekundenlaserpulsen, jeweils fokussiert auf eine Fokalfläche, für eine Auflösung des Augengewebes (21) in einem Fokus (F) im Innern des Augengewebes (21),
**gekennzeichnet durch**,
optische Mittel (13) zur Erzeugung von jeweils **durch** einen der Femtosekundenlaserpulse auf der Fokalfläche gebildeten Pulsbearbeitungsbereichen (E) mit jeweils einer von einer kreisrunden Form abweichenden, im wesentlichen elliptischen oder ovalen Bereichsgrenze (e) auf der Fokalfläche, und
Positionierungsmittel (12) zum Positionieren der jeweils **durch** einen der Femtosekundenlaserpulse auf der Fokalfläche gebildeten elliptischen oder ovalen Pulsbearbeitungsbereiche (E) entlang einer Bearbeitungslinie (s, s*),
wobei die optischen Mittel (13) eingerichtet sind, die jeweils **durch** einen der Femtosekundenlaserpulse auf der Fokalfläche gebildeten elliptischen oder ovalen Pulsbearbeitungsbereiche (E) bezüglich der Bearbeitungslinie (s, s*) gezielt auszurichten.

2. Vorrichtung (1) nach Anspruch 1, **dadurch gekennzeichnet, dass** die optischen Mittel (13) eingerichtet sind, die Pulsbearbeitungsbereiche (E) bezüglich der Bearbeitungslinie (s, s*) so auszurichten, dass eine in einer breiteren Ausdehnung des Pulsbearbeitungsbereichs (E) verlaufende Längsachse (r) jeweils senkrecht zur Bearbeitungslinie (s, s*) liegt.

3. Vorrichtung (1) nach einem der Ansprüche 1 oder 2, **dadurch gekennzeichnet, dass** die optischen Mittel (13) eingerichtet sind, die Pulsbearbeitungsbereiche (E) bezüglich der Bearbeitungslinie (s, s*) so auszurichten, dass eine in einer schmäleren Ausdehnung des Pulsbearbeitungsbereichs (E) verlaufende Querachse (q) jeweils senkrecht zur Bearbeitungslinie (s, s*) liegt

4. Vorrichtung (1) nach einem der Ansprüche 1 bis 3, **gekennzeichnet durch** ein Steuermodul (15) zum Steuern der optischen Mittel (13) derart, dass die optischen Mittel (13) die Pulsbearbeitungsbereiche (E) bezüglich der Bearbeitungslinie (s, s*) entsprechend einem Steuerwert ausrichten.

5. Vorrichtung (1) nach einem der Ansprüche 1 bis 4, **gekennzeichnet durch** ein Steuermodul (15) zum Steuern der optischen Mittel (13) derart, dass die optischen Mittel (13) die Form der Bereichsgrenze (e) der Pulsbearbeitungsbereiche (E) entsprechend einem Steuerwert erzeugen.

6. Vorrichtung (1) nach einem der Ansprüche 1 bis 5, **dadurch gekennzeichnet, dass** die Positionierungsmittel (12) und die optischen Mittel (13) eingerichtet sind, die Pulsbearbeitungsbereiche (E) so zu positionieren respektive zu erzeugen, dass sich nacheinander folgende Pulsbearbeitungsbereiche (E) mindestens teilweise überlappen.

7. Vorrichtung (1) nach einem der Ansprüche 1 bis 6, **dadurch gekennzeichnet, dass** die Bereichsgrenze (e) definiert ist durch einen Grenzwert für einen Intensitätsabfall eines der Femtosekundenlaserpulse auf der Fokalfläche.

8. Vorrichtung (1) nach einem der Ansprüche 1 bis 7, **dadurch gekennzeichnet, dass** die optischen Mittel (13) zur Erzeugung der Pulsbearbeitungsbereiche (E) mindestens eines aus Polarisationsmodulator, Phasenmodulator und Intensitätsmodulator umfassen.

9. Vorrichtung (1) nach einem der Ansprüche 1 bis 8, **dadurch gekennzeichnet, dass** die optischen Mittel (13) zur Erzeugung der Pulsbearbeitungsbereiche (E) mindestens eines aus anamorphotisches Optikmodul, deformierbarer Spiegel, photonischer Kristall, photonische "band-gap" Faser, diffraktives Optikmodul, Linsen-Array, Polarisationsfilter, räumliche Polarisationsplatte, λ-Halbe-Platte, Blende und räumlicher Lichtmodulator umfassen.

10. Vorrichtung (1) nach Anspruch 1, **dadurch gekennzeichnet, dass** die optischen Mittel (13) eingerichtet sind, die jeweils durch einen der Femtosekundenlaserpulse auf der Fokalfläche gebildeten elliptischen oder ovalen Pulsbearbeitungsbereiche (E) bezüglich einer gekrümmten Bearbeitungslinie (s*) gezielt auszurichten.

## Claims

1. Ophthalmological device (1) for generating cuts in the eye tissue (21), comprising:
a light projector (11) for projecting femtosecond laser pulses, respectively focussed onto a focal plane, for dissolving the eye tissue (21) in a focus (F) in the interior of the eye tissue (21),
**characterized by**
optical means (13) for generating pulse processing areas (E), respectively formed on the focal plane by one of the femtosecond laser pulses, each area having a substantially elliptic or oval area boundary (e) on the focal plane, which differs from a circular shape, and
positioning means (12) for positioning the elliptic or oval pulse processing areas (E), respectively formed on the focal plane by one of the femtosecond laser pulses, along a processing line (s, s*),
with the optical means (13) being designed for aligning the elliptic or oval pulse processing areas (E), respectively formed on the focal plane by one of the femtosecond laser pulses, with respect to the processing line (s, s*) in a targeted fashion.

2. Device (1) according to Claim 1, **characterized in that** the optical means (13) are designed for aligning the pulse processing areas (E) with respect to the processing line (s, s*) such that a longitudinal axis (r) running along a broader extent of the pulse processing area (E) is in each case perpendicular to the processing line (s, s*).

3. Device (1) according to one of Claims 1 or 2, **characterized in that** the optical means (13) are designed for aligning the pulse processing areas (E) with respect to the processing line (s, s*) such that a lateral axis (q) running along a thinner extent of the pulse processing area (E) is in each case perpendicular to the processing line (s, s*).

4. Device (1) according to one of Claims 1 to 3, **characterized by** a control module (15) for controlling the optical means (13) such that the optical means (13) align the pulse processing areas (E) with respect to the processing line (s, s*) in accordance with a control value.

5. Device (1) according to one of Claims 1 to 4, **characterized by** a control module (15) for controlling the optical means (13) such that the optical means (13) produce the shape of the area boundary (e) of the pulse processing areas (E) in accordance with a control value.

6. Device (1) according to one of Claims 1 to 5, **characterized in that** the positioning means (12) and the optical means (13) are designed for positioning or producing the pulse processing areas (E) such that respectively successive pulse processing areas (E) overlap at least in part.

7. Device (1) according to one of Claims 1 to 6, **characterized in that** the area boundary (e) is defined by a threshold for an intensity drop of one of the femtosecond laser pulses on the focal plane.

8. Device (1) according to one of Claims 1 to 7, **characterized in that** the optical means (13) for generating the pulse processing areas (E) comprise at least one of the following elements: polarization modulator, phase modulator and intensity modulator.

9. Device (1) according to one of Claims 1 to 8, **characterized in that** the optical means (13) for generating the pulse processing areas (E) comprise at least one of the following elements: anamorphic optic module, deformable mirror, photonic crystal, photonic bandgap fibre, diffractive optic module, lens array, polarization filter, spatial polarization plate, half-wavelength plate, stop and spatial light modulator.

10. Device (1) according to Claim 1, **characterized in that** the optical means (13) are designed for aligning the elliptic or oval pulse processing areas (E), respectively formed on the focal plane by one of the femtosecond laser pulses, with respect to a curved processing line (s*) in a targeted fashion.

## Revendications

1. Appareil ophtalmologique (1) pour générer des coupes dans le tissu oculaire (21), comprenant :
un projecteur de lumière (11) pour projeter des impulsions laser d'une femtoseconde, à chaque fois concentrées sur une surface focale, pour une séparation du tissu oculaire (21) dans un foyer (F) à l'intérieur du tissu oculaire (21),
**caractérisé par**
des moyens optiques (13) pour générer des zones de traitement par impulsions (E) respectivement formées sur la surface focale par l'une des impulsions laser d'une femtoseconde, à chaque fois avec une limite de zone (e) différente d'une forme circulaire, essentiellement elliptique ou ovale sur la surface focale, et
des moyens de positionnement (12) pour positionner les zones de traitement par impulsions (E) elliptiques ou ovales respectivement formées sur la surface focale par l'une des impulsions laser d'une femtoseconde le long d'une ligne de traitement (s, s*),
les moyens optiques (13) étant conçus pour orienter de manière ciblée les zones de traitement par impulsions (E) elliptiques ou ovales respectivement formées sur la surface focale par l'une des impulsions laser d'une femtoseconde par rapport à la ligne de traitement (s, s*).

2. Appareil (1) selon la revendication 1, **caractérisé en ce que** les moyens optiques (13) sont conçus pour orienter les zones de traitement par impulsions (E) par rapport à la ligne de traitement (s, s*) de telle sorte qu'un axe longitudinal (r) qui s'étend dans une extension plus large de la zone de traitement par impulsions (E) est à chaque fois perpendiculaire à la ligne de traitement (s, s*).

3. Appareil (1) selon la revendication 1 ou 2, **caractérisé en ce que** les moyens optiques (13) sont conçus pour orienter les zones de traitement par impulsions (E) par rapport à la ligne de traitement (s, s*) de telle sorte qu'un axe transversal (q) qui s'étend dans une extension plus étroite de la zone de traitement par impulsions (E) est à chaque fois perpendiculaire à la ligne de traitement (s, s*).

4. Appareil (1) selon l'une des revendications 1 à 3, **caractérisé par** un module de commande (15) pour commander les moyens optiques (13) de telle sorte que les moyens optiques (13) orientent les zones de traitement par impulsions (E) par rapport à la ligne de traitement (s, s*) en fonction d'une valeur de commande.

5. Appareil (1) selon l'une des revendications 1 à 4, **caractérisé par** un module de commande (15) pour commander les moyens optiques (13) de telle sorte que les moyens optiques (13) génèrent la forme d'une limite de zone (e) des zones de traitement par impulsions (E) en fonction d'une valeur de commande.

6. Appareil (1) selon l'une des revendications 1 à 5, **caractérisé en ce que** les moyens de positionnement (12) et les moyens optiques (13) sont conçus pour positionner, respectivement pour générer, les zones de traitement par impulsions (E) de telle sorte que les zones de traitement par impulsions (E) successives se chevauchent au moins partiellement.

7. Appareil (1) selon l'une des revendications 1 à 6, **caractérisé en ce que** la limite de zone (e) est définie par une valeur limite d'une chute de l'intensité de l'une des impulsions laser d'une femtoseconde sur la surface focale.

8. Appareil (1) selon l'une des revendications 1 à 7, **caractérisé en ce que** les moyens optiques (13) comprennent, pour générer les zones de traitement par impulsions (E), au moins l'un des éléments suivants : modulateur de polarisation, modulateur de phase ou modulateur d'intensité pour générer les zones de traitement par impulsions (E).

9. Appareil (1) selon l'une des revendications 1 à 8, **caractérisé en ce que** les moyens optiques (13) comprennent, pour générer les zones de traitement par impulsions (E), au moins l'un des éléments suivants : module optique, miroir déformable, cristal photonique, fibre à GAP photonique, module optique diffractif, réseau de lentilles, filtre à polarisation, plaque de polarisation dans l'espace, demi plaque λ, obturateur et modulateur de lumière dans l'espace.

10. Appareil (1) selon la revendication 1, **caractérisé en ce que** les moyens optiques (13) sont conçus pour orienter de manière ciblée les zones de traitement par impulsions (E) elliptiques ou ovales respectivement formées sur la surface focale par l'une des impulsions laser d'une femtoseconde par rapport à une ligne de traitement courbe (s*).
